# EUROPÄISCHE PATENTSCHRIFT

(11) **EP 0 387 605 B1**
(45) Veröffentlichungstag und Bekanntmachung des Hinweises auf die Patenterteilung: **30.03.1994**
(21) Anmeldenummer: 90103858.8
(22) Anmeldetag: 28.02.1990
(51) Int. Cl.: C07C 227/32, C07C 229/22, C07C 213/00, C07C 215/08, C07D 207/08

(54) **Verfahren zur stereoselektiven Herstellung von optisch aktiven S,S- oder R,R-beta-Aminoalkoholen**
Process for the stereoselective preparation of optically active S,S or R,R-beta-aminoalcohols
Procédé pour la préparation stéréosélective de S,S ou R,R-bêta-aminoalcools optiquement actifs

(30) Priorität: 15.03.1989 DE 3908426
(43) Veröffentlichungstag der Anmeldung: 19.09.1990
(73) Patentinhaber: BAYER AG, 51368 Leverkusen (DE)
(72) Erfinder: Reetz, Manfred Theodor, Prof. Dr., D-3550 Marburg (DE); Drewes, Mark Wilhelm, Dr., D-4018 Langenfeld (DE); Lennick, Klaus, D-3550 Marburg (DE)

(56) Entgegenhaltungen:
- EP-A- 0 288 764
- JOURNAL OF THE CHEMICAL SOCIETY, CHEMICAL COMMUNICATIONS, Nr. 18, September 1989, Seiten 1474,1475, Letchworth, GB; M.T. REETZ et al: "A simple synthetic route to statine and statine analogues"
- ANGEWANDTE CHEMIE, INTERNATIONAL EDITION, Band 99, Nr. 11, 1987, Seiten 1186-1188, Weinheim, DE; M.T. REETZ et al: "Stereoselektive Synthese von beta-Aminoalkoholen aus optisch aktiven alpha-Aminosäuren"
- ANGEWANDTE CHEMIE, INTERNATIONAL EDITION, Band 100, Nr. 3, 1988, Seiten 414,415, Weinheim, DE; P. RADDATZ et al: "Reduktion mit Hefezellen, der Schluesselschritt einer effizienten Synthese von (3S,4S)-4-Amino-3-hydroxypentansäuren"
- PATENT ABSTRACTS OF JAPAN, Band 6, Nr. 3 (C-86)(881), 9.Januar 1982; & JP - A - 56128740
- TETRAHEDRON, (INCL. TETRAHEDRON REPORTS), Band 42, Nr. 7, 1986, Seiten 2117-2120, Oxford, GB; G. BETTONI et al: "Synthesis of rigid dopamine congeners: Cis and trans 2-(p-methoxyphenyl)-3-methylmorpholine"
- JUSTUS LIEBIGS ANNALEN DER CHEMIE, 1975, Seiten 2245-2250, Weinheim, DE; R. STEULMANN et al: "Synthesen der (3S,4S)-4-Amino-3-hydroxy-6-methylheptansäure und einiger Derivate"

## Beschreibung

Die Erfindung betrifft ein neues Verfahren zur stereoselektiven Herstellung von optisch aktiven (S,S)- oder (R,R)-β-Aminoalkoholen, insbesondere zur Herstellung von Statin und Statin-Analogen.

Optisch aktive β-Aminoalkohole sind wichtige Zwischenprodukte für die Herstellung biologisch aktiver Verbindungen und zwar sowohl natürlich vorkommender als auch synthetisch hergestellter Wirkstoffe, Besonderes Interesse haben in der Fachwelt Statin ((3S, 4S)-4-Amino-3-hydroxy-6-methylheptansäure) und seine Analoga gefunden. Statin und Statin-Analoga sind zentrale Bausteine vieler hochwirksamer spezifischer Aspartylprotease-Inhibitoren, u.a. auch von Renin-Inhibitoren, die als blutdrucksenkende Medikamente interessant sind. Kinetische Studien haben gezeigt, daß die Hydroxygruppe in Statinen S-konfiguriert sein muß, die Kohlenstoffkette an C-4 jedoch chemisch modifiziert werden kann.

Infolge der interessanten biologischen Wirkung der optisch aktiven β-Aminoalkohole, insbesondere des Statins und seiner Analoga, hat es nicht an Versuchen gefehlt, für diese optisch aktiven β-Aminoalkohole wirtschaftliche Verfahren zur Herstellung aufzufinden (siehe (a) J. Org. Chem. 43 (1978) 3624; (b) Liebigs Ann. Chem. 1975, 2245; (c) J. Med. Chem. 30 (1987) 374; (d) J. Org. Chem. 47 (1982) 1981; (e) Tetrahedron Lett. 26 (1985) 2973; (f) J. Chem. Soc. Perkin Trans. I 1985, 1985; (g) J. Chem. Soc. Chem. Commun. 1987, 311; (h) Angew. Chem. 100 (3) 1988, 414; (j) EP-A 288 764; (k) J. Org. Chem. 53 (4), (1989), 869). Diese bekannten Verfahren zur Herstellung von Statin und Statin-Analoga erfüllen aber nicht die an eine technisch nutzbare Statinsynthese gestellten Anforderungen, nämlich von leicht zugänglichen Ausgangsverbindungen auszugehen und/oder leicht zugängliche Reagentien zu verwenden und stereoselektiv die erforderliche 3S, 4S-Konfiguration zu liefern.

Überraschenderweise wurde gefunden, daß man optisch aktive β-Aminoslkohole wie Statin und seine Analoga aus gut zugänglichen optisch aktiven Verbindungen in guten Ausbeuten und hoher Stereoselektivität in der gewünschten S,S- oder der R,R-Konfiguration erhält, wenn man optisch aktive (S)-α-Aminoketone bzw. (R)-α-Aminoketone, deren Aminogruppen nicht mit den üblichen Schutzgruppen sondern mit bestimmten anderen Gruppen maskiert sind, mit komplexen Metallhydriden reduziert. Es wurde gefunden, daß die Reduktion der in bestimmter Weise am Stickstoff substituierten β-Aminoalkohole mit komplexen Metallhydriden stereoselektiv zu den optisch aktiven β-Aminoalkoholen führt, deren Bildung nicht-chelat-kontrolliert erfolgt, d.h. die Reduktion liefert im Falle der optisch aktiven (S)-α-Aminoketone in hoher Stereoselektivität die optisch aktiven (S,S)-β-Aminoalkohole und im Falle der optisch aktiven (R)-α-Aminoketone die optisch aktiven (R,R)-β-Aminoalkohole.

Das heißt, es wurde gefunden, daß die Reduktion der in bestimmter Weise substituierten α-Aminoketone mit komplexen Metallhydriden nicht nur selektiv unter Bildung irgendeines der beiden möglichen Diastereomere (S,S)- oder (S,R)- bzw. (R,R)- oder (R,S)- verläuft, sondern daß in Abhängigkeit von der Konfiguration des α-Aminoketons gezielt Diastereomere mit der (S,S)-Konfiguration bzw. Diastereomere mit der (R,R)-Konfiguration erhalten werden.

Die Erfindung betrifft daher ein Verfahren zur stereoselektiven Herstellung von (S,S)- oder (R,R)-β-Aminoalkoholen der Formeln 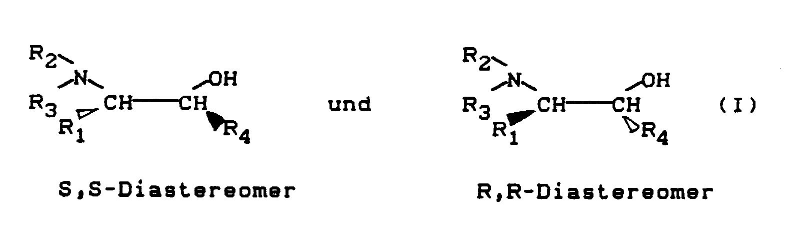
in denen
- R₁: für einen gegebenenfalls substituierten Alkyl-, Cycloalkyl-, Alkenyl-, Aralkyl-, Aryl- oder Heteroaryl-Rest steht,
- R₂ und R₃: unabhängig voneinander eine gegebenenfalls substituierte Aralkyl-Gruppe vorzugsweise eine gegebenenfalls substituierte Benzylgruppe, bedeuten,
- R₄: für einen gegebenenfalls substituierten Alkyl-, Alkenyl-, Alkinyl-, Aralkyl-, Aryl- oder Heteroaryl-Rest steht,
das dadurch gekennzeichnet ist, daß man optisch aktive α-Aminoketone der Formeln 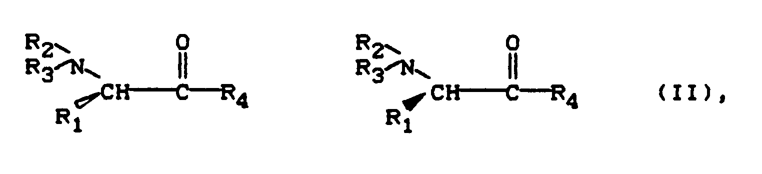
in denen
R₁, R₂, R₃ und R₄ die unter Formel (I) angegebene Bedeutung haben, mit komplexen Metallhydriden reduziert.

Als komplexe Metallhydride seien vor allem Lithiumaluminiumhydrid, Natrium-, Kalium- oder Zinkborhydrid oder Natriumcyanborhydrid oder Natriumborhydrid genannt; bevorzugt wird Natriumborhydrid verwendet.

Die Reduktion wird in Wasser oder polaren organischen Lösungsmitteln, vorzugsweise in niederen Alkoholen wie Methanol, Glykolethern wie Glykolmonomethylether, Diethylenglykolmonomethylether, Diethylenglykoldimethylether, Triethylenglykoldimethylether oder Dimethylsulfoxid oder Dimethylformamid bei Temperaturen von -50 bis +30°C vorgenommen.

Die komplexen Metallhydride werden in einer für die Reduktion von Ketonen üblichen Menge eingesetzt, nämlich von 1 bis 1,5 Mol Metallhydrid je Mol Keto-Gruppe.

Die β-Aminoalkohole der Formeln (I) und α-Aminoketone der Formeln (II) werden im Rahmen der vorliegenden Erfindung vereinfachend als peralkylierte Aminoalkohole bzw. Aminoketone bezeichnet. Weil die Benzylgruppen besonders einfach abspaltbar sind, sind die perbenzylierten Aminoalkohole der Formeln (I) und die perbenzylierten Aminoketone der Formeln (II) bevorzugt.

Für R₁ seien als gegebenenfalls substituierte Alkylreste beispielsweise genannt; C₁-C₁₂-Alkylreste wie der Methyl-, Ethyl-, n- und iso-Propyl-, n-Butyl-, sec.-Butyl-, iso- Butyl-, Amyl- und Hexyl-Rest; als Substituenten kommen für die Alkylreste vorzugsweise Cycloalkylreste wie der Cyclohexylrest, die Amino-, Hydroxy-, Benzyloxy-, Benzylmercapto-, Carbonamido- oder eine gegebenenfalls am N substituierte Imidazolyl-4- oder Indolyl-3-Gruppe in Betracht. Als substituierte Alkylreste seien beispielsweise genannt: der 1-Hydroxyethyl-, 2-Alkoxycarbonylethyl-, 3-Alkoxycarbonylpropyl-, tert.-Butoxy-methyl- und der Hydroxymethyl-Rest; ferner der Benzyloxymethyl-, 1-Benzyloxyethyl-, Carbamoylmethyl-, 2-Carbamoylethyl-, 3-(N,N- Dibenzylamino)-propyl-, 4-(N,N-Dibenzylamino)-butyl-, der (Imidazolyl-4)-methyl-, und der (Indolyl-3)-methyl- Rest; als gegebenenfalls substituierte Cycloalkylreste der Cyclohexyl-, Methylcyclohexyl- und der Tetrahydronaphthyl-2-Rest; als gegebenenfalls substituierte Alkenylreste beispielsweise der Vinyl-Rest.

Als gegebenenfalls substituierte Aralkylreste seien beispielsweise genannt: der Benzylrest und durch OH, C₁-C₄-Alkyl, C₁-C₄-Alkoxy, Benzyloxy oder Halogenatome substituierte Benzylreste, wie der 4-Hydroxybenzyl-, der 2- und 4-Methylbenzylrest, der 4-Methoxybenzyl- und der 4-Benzyloxybenzyl-Rest; als gegebenenfalls substituierter Heteroarylrest der Indolyl-3-Rest.

Als gegebenenfalls substituierte Arylreste seien beispielsweise genannt der Phenyl- und der Naphthylrest und durch OH-, C₁-C₄-Alkoxy- und C₁-C₄-Alkylgruppen substituierte Phenylreste wie der 4-Hydroxyphenylrest, der 4-Methoxyphenylrest und der 2-Methyl- und 4-Methylphenylrest.

Für R₂ und R₃ seien als gegebenenfalls substituierte Benzylreste durch C₁-C₄-Alkylgruppen, C₁-C₄-Alkoxy- und Benzyloxygruppen oder durch Halogenatome substituierten Benzyl- und Phenylen-(1,2)-bis-methylen-Reste genannt; zum Beispiel der 2- und 4-Methylbenzylrest, der 2- und 4-Methoxybenzylrest und der α-Methylbenzylrest.

Für R₄ seien als Alkylreste C₁-C₁₂-Alkylreste, als Alkenyl-Reste vorzugsweise der Vinyl- und Allylrest, als Alkinyl-Reste der Propargylrest, als Aralkylreste vorzugsweise Benzyl- und α-Methylnaphthylreste und als Heteroaryl-Reste vorzugsweise der Thienyl-2-Rest genannt.

Für R₄ seien als Substituenten für die Alkyl-, Alkenyl-, Alkinyl-, Aralkyl-, Phenyl- und Heteroaryl-Reste beispielsweise geschützte Hydroxy-, Mercapto-, Amino- und Carboxylgruppen, ferner Carbonester-, Sulfoxid-, Sulfon- und Cyan-Gruppen, genannt.

Als Beispiele für substituierte Alkylreste seien beispielsweise der Cyanmethyl- und Carbalkoxymethyl-Rest genannt.

Aus den β-Aminoalkoholen der Formeln (I) lassen sich die Benzylgruppen in an sich bekannter Weise durch katalytische Hydrierung (Hydrogenolyse) abspalten. Die Hydrogenolyse wird vorzugsweise mit Pd-Schwarz/Kohle unter Verwendung von Ameisensäure als Wasserstoffdonator vorgenommen. Die bei der Hydrogenolyse zunächst gebildeten Formamide werden anschließend entweder alkalisch durch Zugabe von Basen oder aber - bevorzugt - sauer durch Zugabe verdünnter Salzsäure in Gegenwart von Kieselgel hydrolysiert.

Die für das erfindungsgemäße Verfahren als Ausgangsverbindungen benötigten optisch aktiven N-disubstituierten α-Aminoketone werden durch Alkylieren, vorzugsweise Benzylieren, der entsprechenden optisch aktiven Aminosäuren, Überführen der am Stickstoff peralkylierten Aminosäuren in bekannter weise, z.B. mittels Dicyclohexylcarbodiimid oder Diethylphosphorylcyanid und N,O-Dimethylhydroxylaminhydrochlorid, in die entsprechenden optisch aktiven N-Methoxy-N-methyl-aminosäureamide oder mittels Carbonyldiimidazol, in die entsprechenden optisch aktiven Aminosäureimidazolide und Umsetzen der Aminosäureamide bzw. -imidazolide mit metallorganischen Verbindungen wie Lithium-organischen Verbindungen, Grignardverbindungen oder Enolaten entsprechend dem folgenden Reaktionsschema erhalten.

Die optisch aktiven α-Aminoketone leiten sich vorzugsweise von optisch aktiven Aminosäuren wie Alanin, Aminobuttersäure, Valin, Norvalin, Leucin, Isoleucin, Terleucin, Phenylglycin, Phenylalanin, Naphthylalanin, Cyclohexylglycin, Cyclohexylalanin, Tyrosin, Tryptophan, Threonin, Serin, Asparaginsäure, Glutaminsäure, Ornithin oder Lysin ab. 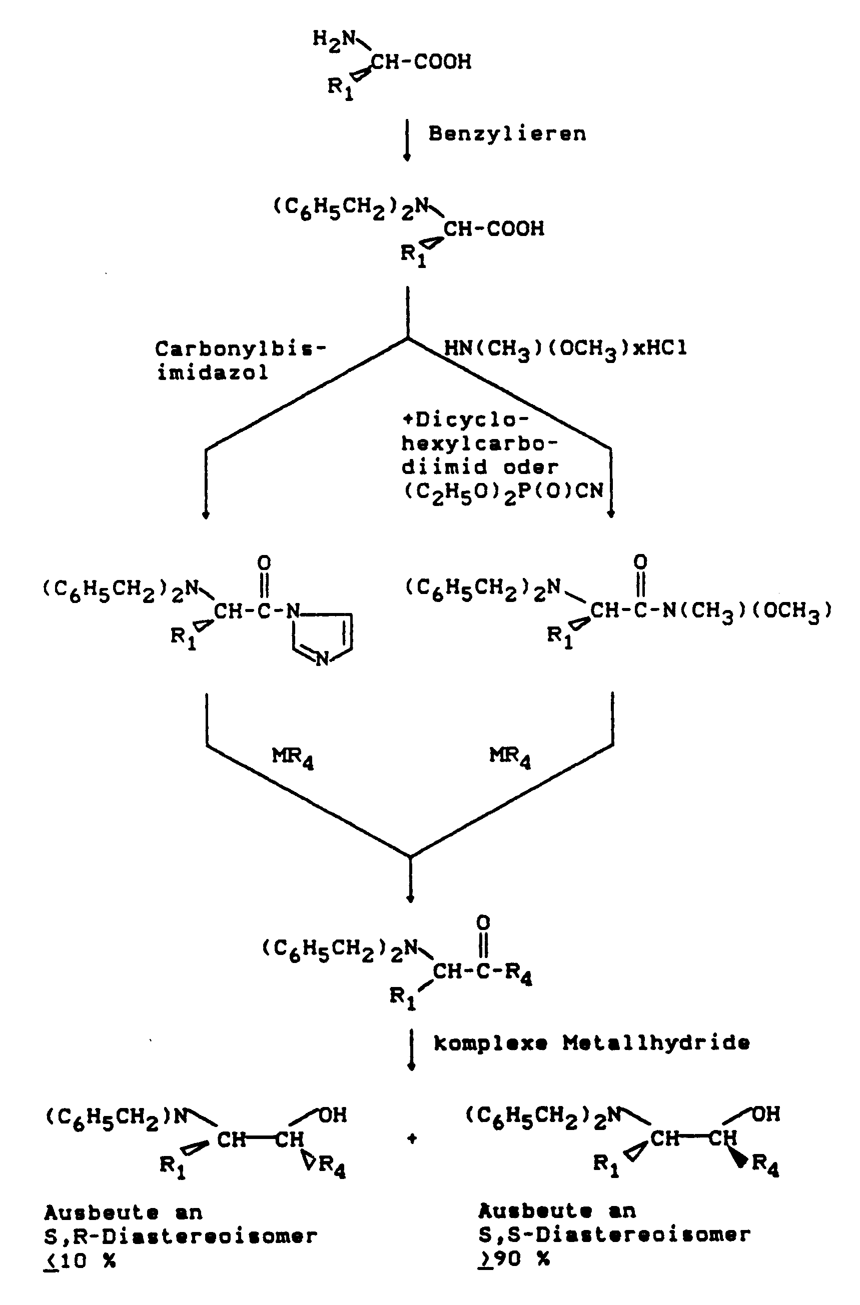
Am N-peralkylierte α-Aminosäuren und ihre Herstellung sind bekannt (siehe Beilstein E IV, Bd. 12 S. 2287, 2295-2297; System-Nr. 1699). Die am N-peralkylierten, optisch aktiven α-Aminosäuren werden analog durch Alkylieren entsprechender optisch aktiver α-Aminosäuren, zum Beispiel natürlich vorkommender Aminosäuren, hergestellt.

Als optisch aktive α-Aminosäuren werden bevorzugt die in der Natur vorkommenden optisch aktiven α-Aminosäuren verwendet.

Die Umsetzung der optisch aktiven, am N-peralkylierten Aminosäuren zu den am Amidstickstoff disubstituierten Säureamiden wird in bekannter Weise durch Umsetzung der am Stickstoff peralkylierten Aminosäuren mit N,O-Dimethylhydroxylaminhydrochlorid in Gegenwart von Dicyclohexylcarbodiimid oder Diethylphosphorylcyanid (siehe z.B. Tetrahedron Lett. 1981, 3815) oder N,N-Carbonyldiimidazol (siehe z.B. Liebigs Ann. Chem. 655, (1962), 90) vorgenommen.

Die Umsetzung dieser Säureamide mit metallorganischen Verbindungen erfolgt ebenfalls in bekannter Weise (siehe z.B. Tetrahedron Lett. 1980, 3815; Liebigs Ann. Chem. 655 (1962) 90; J. Org. Chem. 53, (1988) 873; Angew. Chem. 100 (1988) 414).

### Beispiel 1

a) Die Lösung von 2,0 g (= 4,82 mmol) S-4-(N,N-Dibenzylamino)-3-oxo-5-phenylpentansäureethylester in 10 ml Methanol wird bei -20°C unter Rühren portionsweise mit 270 mg (= 7,2 mmol) NaBH₄ versetzt. Nach 3-stündigem Rühren wird das Reaktionsgemisch mit Wasser versetzt und anschließend mit Ether extrahiert. Die vereinigten Etherextrakte werden über wasserfreiem Natriumsulfat getrocknet und anschließend vom Ether befreit.
   Gemäß ¹³C-NMR-Spektrum weist das Rohprodukt (der Rückstand) ein Diastereomerenverhältnis von >95:<5 auf.
   Nach chromatographischer Reinigung des Rohproduktes an Kieselgel (Laufmittel:Petrolether/Essigester 20:1) werden 1,6 g (= 80 % der Theorie) (3S,4S)-4-(N,N-Dibenzylamino)-3-hydroxy-5-phenylpentansäureethylester als farbloses Öl erhalten. Enantiomerenreinheit des Esters: 93 %.
   Der als Ausgangsverbindung verwendete S-4-(N,N-Dibenzylamino)-3-oxo-5-phenylpentansäureethylester war wie folgt erhalten worden:
b) Die Lösung von 10 g (= 60,5 mmol) S-Phenylalanin und 32,4 g (= 240 mmol) K₂CO₃ in 100 ml Wasser wurde bei 100°C unter Rühren langsam mit 31,2 g (= 181 mmol) Benzylbromid versetzt. Das Reaktionsgemisch wurde noch 1 Stunde bei Rückflußtemparatur gerührt, dann abgekühlt und mit 4,8 g (120 mmol) KOH behandelt. Nach Zugabe von 200 ml Dioxan und 220 ml Methanol wurde die Mischung etwa 6 Stunden auf Rückflußtemperatur erhitzt, dann mit 100 ml Wasser versetzt und mit 450 ml Ether extrahiert. Die wäßrige Phase wurde nach dem Ansäuern (auf einen pH-Wert von 4-5) mit 2N Salzsäure noch dreimal mit je 150 ml Ether extrahiert. Die vereinigten Etherextrakte wurden mit Kochsalzlösung gewaschen, dann über Natriumsulfat getrocknet und im Vakuum vom Lösungsmittel befreit. Der Rückstand wurde aus Ether/Petrolether (1:9) umkristallisiert; Ausbeute: 15,1 g (= 72 % der Theorie) S-2-(N,N-Dibenzylamino)-3-phenylpropionsäure. Fp.: 54°C.
c) Die Lösung von 2,5 g (= 7,24 mmol) S-2-(N,N-Dibenzylamino)-3-phenylpropionsäure in 25 ml Tetrahydrofuran wurde bei Raumtemperatur unter Rühren portionsweise mit 1,3 g (= 7,9 mmol) N,N-Carbonyldiimidazol versetzt. Nach dem Abklingen der Gisentwicklung wurde das Reaktionsgemisch 1 Stunde bei Raumtemperatur gerührt.
   Das entstandene S-2-(N,N-Dibenzylamino)-3-phenylpropionsäureimidazolid wurde nicht isoliert, sondern das erhaltene Reaktionsgemisch unmittelbar bei 0° unter Rühren langsam mit der Hälfte einer aus 1,9 g (=14,5 mmol) frisch destilliertem Malonsäuremonoethylester und 29 mmol Isopropylmagnesiumchlorid bereiteten Lösung des Magnesium-Enolats des Malonsäuremonoethylesters in Tetrahydrofuran versetzt. Das Reaktionsgemisch wurde auf 45°C erwärmt und mit der zweiten Hälfte der Magnesium-Enolat-Lösung, versetzt. Nach 12-stündigem Rühren wurde die Reaktionsmischung auf kalte 1N Salzsäure-Lösung gegeben und dieses wäßrige Gemisch mit Ether extrahiert. Die organische Phase wurde mit Kochsalzlösung gewaschen und über Natriumsulfat getrocknet. Der nach dem Entfernen des Lösungsmittels verbleibende Rückstand, ein gelbes Öl, wurde durch Chromatographie an Kieselgel (Petrolether/Essigester 50:1) gereinigt; Ausbeute 2,34 g (= 78 % der Theorie) S-4-(N,N-Dibenzylamino)-3-oxo-5-phenylpentansäureethylester als farbloses Öl.

### Beispiel 2

a) Es wurde wie in Beispiel 1a) beschrieben verfahren, nur wurden anstelle der 2,0 g S-4-(N,N-Dibenzylamino)-3-oxo-5-phenylpentansäureethylester 3,5 g (= 9,2 mmol) S-4-(N,N-Dibenzylamino)-3-oxo-6-methylheptansäureethylester eingesetzt.
   Es werden 2,93 g (= 83 % der Theorie) (3S,4S)-4-(N,N-Dibenzylamino)-3-hydroxy-6-methylheptansäureethylester (Fp.: 74-75°C) erhalten. Enantiomerenreinheit des Esters: >99,5 %.
   Der als Ausgangsverbindung verwendete (N,N-Dibenzylamino)-3-oxo-6-methylheptansäureethylester war wie folgt erhalten worden:
b) 30 g (= 228 mmol) S-Leucin wurden nach der in Beispiel 1b) beschriebenen Arbeitsweise benzyliert. Es wurden 49,5 g (= 70 % der Theorie) S-2-(N,N-Dibenzylamino)-4-methylpentansäure erhalten; Fp.: 84-88°C.
c) Entsprechend der in Beispiel 1c) beschriebenen Arbeitsweise wurden 10,0 g (= 32 mmol) S-2-(N,N-Dibenzylamino)-4-methylpentansäure durch Überführung in das Imidazolid und Umsetzung des Imidazolides mit dem Magnesium-Enolat des Malonsäuremonoethylesters in den S-4-(N,N-Dibenzylamino)-3-oxo-6-methylheptansäureethylester überführt; Ausbeute 9,19 g (= 78 % der Theorie).
d) Dibenzylierung des (3S,4S)-4-(Dibenzylamino)-3-hydroxy-6-methyl-heptansäureethylesters:
   200 mg Palladium-Schwarz in 20 ml Methanol und 3 ml Ameisensäure wurden tropfenweise mit 2,7 g (= 7,05 mmol) (3S,4S)-4-(Dibenzylamino)-3-hydroxy-6-methylheptansäureethylester in 2 ml Methanol versetzt. Die Mischung wurde zunächst 12 Stunden bei Raumtemperatur, dann weitere 4 Stunden bei 50°C gerührt. Der Katalysator wurde abgesaugt und das Filtrat im Vakuum eingeengt. Der Rückstand wurde durch Chromatographie an Kieselgel (Laufmittel: Chloroform/Methanol 19:1) gereinigt; es wurden 1,28 g (= 89 % der Theorie) (3S,4S)-4-Amino-3-hydroxy-6-methyl-heptansäureethylester erhalten.

### Beispiel 3

a) Es wurde wie in Beispiel 1 beschrieben gearbeitet, nur wurde anstelle der 2,0 g S-4-(N,N-Dibenzylamino)-3-oxo-5-phenylpentansäureethylester 0,40 g (= 0,95 mmol) S-4-(N,N-Dibenzylamino)-3-oxo-5-cyclohexylpentansäureethylester eingesetzt. Es wurden 0,34 g (= 84 % der Theorie) (3S,4S)-4-(N,N-Dibenzylamino)-3-hydroxy-5-cyclohexylpentansäureethylester erhalten; Enantiomerenreinheit des Esters: >99,5 %.
   Der als Ausgangsverbindung verwendete S-4-(N,N-Dibenzylamino)-3-oxo-5-cyclohexylpentansäureethylester war wie folgt erhalten worden:
b) 10 g (= 58 mmol) S-2-Amino-3-cyclohexylpropionsäure wurden entsprechend der in Beispiel 1b) beschriebenen Arbeitsweise benzyliert. Es wurden 14,1 g (= 69 % der Theorie) S-2-(N,N-Dibenzylamino)-3-cyclohexylpropionsäure erhalten; Fp.: 41°C.
c) Entsprechend der in Beispiel 1c) beschriebenen Arbeitsweise wurden 0,50 g (= 1,42 mmol) S-2-(N,N-Dibenzylamino)-3-cyclohexylpropionsäure durch Überführung in das Imidazolid und Umsetzung des Imidazolides mit dem Magnesium-Enolat des Malonsäurehalbesters in den S-4-(N,N-Dibenzylamino)-3-oxo-5-cyclohexylpentansäureethylester überführt; Ausbeute 0,422 g (= 71 % der Theorie).

### Beispiel 4

a) Die Lösung von 184 mg (= 0,27 mmol) S-3-(N,N-Dibenzylamino)-4-phenylbutan-2-on in 4 ml Methanol wird unter Rühren bei -15°C mit 21 mg (= 0,27 mmol) NaBH₄ versetzt. Nach 3-stündigem Rühren wird das Reaktionsgemisch wie in Beispiel 1a) beschrieben aufgearbeitet. Es werden 162 mg (= 88 % der Theorie) (2S,3S)-3-(N,N-Dibenzylamino)-4-phenylbutan-2-ol erhalten. Das Diastereomerenverhältnis im Rohprodukt beträgt 95:5; die Enantiomerenreinheit des nach der chromatographischen Reinigung des Rohproduktes erhaltenen reinen Produktes beträgt 91 %.
   Das als Ausgangsverbindung verwendete S-3-(N,N-Dibenzylamino)-4-phenylbutan-2-on war wie folgt erhalten worden:
b) S-2-Amino-3-phenylpropionsäure wurde wie im Beispiel 1b) beschrieben benzyliert.
c₁) Die Lösung von 200 mg (= 0,58 mmol) S-2-(N,N-Dibenzylamino)-3-phenylpropionsäure und 117 mg (1,6 mmol) Triethylamin in 200 ml Dichlormethan wurde bei Raumtemperatur unter Rühren mit 68 mg (= 0,69 mmol) N,O-Dimethylhydroxylaminhydrochlorid versetzt. Die Mischung wurde unter Rühren tropfenweise mit 198 mg (= 1,6 mmol) Diethylphosphorylcyanid versetzt. Das Reaktionsgemisch wurde 12 Stunden bei Raumtemperatur gerührt, dann in Wasser eingerührt und das wäßrige Gemisch mit Ether extrahiert. Die vereinigten Etherextrakte wurden nacheinander mit Natriumbicarbonat- und wäßriger 1 %iger HCl-Lösung gewaschen und anschließend über Natriumsulfat getrocknet. Nach dem Entfernen des Lösungsmittels wurde der Rückstand durch Chromatographie an Kieselgel (Laufmittel: Petrolether/ Essigester 20:1) gereinigt. Es wurden 163 mg (= 72 % der Theorie) S-2-(N,N-Dibenzylamino)-N-methoxy-N-methyl-3-phenylpropionsäureamid erhalten; Fp.: 56°C.
c₂) Die Lösung von 200 mg (= 0,52 mmol) S-2-(N,N-Dibenzylamino)-N-methoxy-N-methyl-3-phenylpropionsäureamid in 5 ml trockenem Tetrahydrofuran wurde unter Rühren in einer Inertgasatmosphäre bei 0°C tropfenweise mit 0,56 mmol Methyllithium versetzt. Das Reaktionsgemisch wurde 1 Stunde gerührt, dann in Wasser eingerührt und das wäßrige Gemisch mit Ether extrahiert. Die vereinigten Etherextrakte wurden über Natriumsulfat getrocknet; der nach dem Entfernen des Ethers verbleibende Rückstand wurde durch Chromatographie an Kieselgel (Laufmittel: Petrolether/ Essigester 4:1) gereinigt; es wurden 157 mg (= 89 % der Theorie) S-3-(N,N-Dibenzylamino)-4-phenylbutan-2-on erhalten.

### Beispiel 5

a) Es wurde wie in Beispiel 4a) beschrieben gearbeitet, nur wurde anstelle der 184 mg S-3-(N,N-Dibenzylamino)-4-phenylbutan-2-on 200 mg (= 0,65 mmol) S-3-(N,N-Dibenzylamino)-5-methyl-pentan-2-on verwendet.
   Es wurden 180 mg (= 90 % der Theorie) (2S,3S)-3-(N,N-Dibenzylamino)-2-hydroxy-5-methylpentan erhalten. Das Diastereomerenverhältnis im Rohprodukt betrug 94:6; die Enantiomerenreinheit des an Kieselgel gereinigten Produktes betrug >99 %.
   Das als Ausgangsverbindung eingesetzte S-3-(N,N-Dibenzylamino)-5-methyl-pentan-2-on war wie folgt erhalten worden:
b) S-2-Amino-3-methyl-butancarbonsäure wurde nach der in Beispiel 1b) beschriebenen Arbeitsweise benzyliert.
c₁) Die Lösung von 180 mg (= 0,58 mmol) S-2-(N,N-Dibenzylamino-3-methyl-butansäure, 132 mg (= 0,64 mmol) Dicyclohexylcarbodiimid und einer katalytischen Menge 4-Dimethylaminopyridin in 200 ml Dichlormethan wurde 1 Stunde bei Raumtemperatur gerührt. Dann wurde sie unter Rühren mit einem Gemisch aus 68 mg (= 0,64 mmol) N,O-Dimethylhydroxylaminhydrochlorid und 129 mg (= 1,28 mmol) Triethylamin in 3 ml Dichlormethan versetzt und das Gemisch 22 Stunden bei Raumtemperatur gerührt. Das Gemisch wird in Wasser gegeben, das wäßrige Gemisch mit Dichlormethan extrahiert und die vereinigten Dichlormethanextrakte nach dem Trocknen vom Lösungsmittel befreit. Es wurden 142 mg (= 69 % der Theorie) S-2-(N,N-Dibenzylamino)-N-methoxy-N-methyl-3-methylbutansäureamid erhalten.
c₂) 300 mg (= 0,84 mmol) S-2-(N,N-Dibenzylamino)-N-methoxy-N-methyl-3-methyl-butansäureamid wurden durch Umsetzung mit Methylmagnesiumiodid (in Form einer Lösung in Tetrahydrofuran) nach der in Beispiel 4c₂) für die Umsetzung von S-2-(N,N-Dibenzylamino)-N-methoxy-N-methyl-3-phenylpropionsäureamid mit Methyllithium beschriebenen Arbeitsweise zum S-3-(N,N-Dibenzylamino)-4-methyl-butan-2-on umgesetzt. Ausbeute 162 mg (= 92 % der Theorie).

## Patentansprüche

1. Verfahren zur stereoselektiven Herstellung von (S,S)- oder (R,R)-β-Aminoalkoholen der Formeln 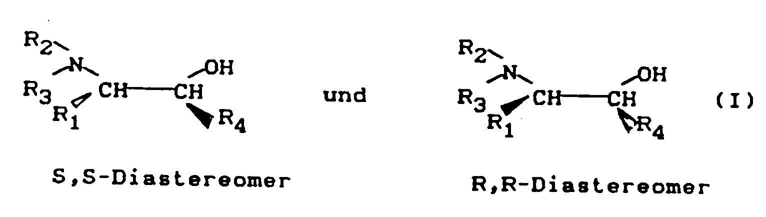 in denen
R₁ für einen gegebenenfalls substituierten Alkyl-, Cycloalkl-, Alkenyl-, Aralkyl-, Aryl- oder Heteroaryl-Rest steht,
R₂ und R₃ unabhängig voneinander eine gegebenenfalls substituierte Aralkyl-Gruppe bedeuten und
R₄ für einen gegebenenfalls substituierten Alkyl-, Alkenyl-, Alkinyl-, Aralkyl-, Aryl- oder Heteroaryl-Rest steht,
dadurch gekennzeichnet, daß man optisch aktive α-Aminoketone der Formeln 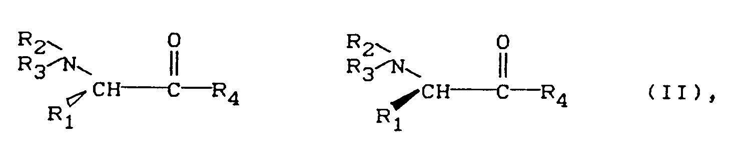 in denen
R₁, R₂, R₃ und R₄ die unter Formel (I) angegebene Bedeutung haben, mit komplexen Metallhydriden reduziert.

2. Verfahren nach Anspruch 1, dadurch gekennzeichnet, daß R₂ und R₃ unahängig voneinander eine gegebenenfalls substituierte Benzylgruppe bedeuten.

3. Verfahren nach Anspruch 1, dadurch gekennzeichnet, daß R₂ und R₃ unsubstituierte Benzylreste sind.

4. Verfahren nach einem der Ansprüche 1, 2 oder 3 dadurch gekennzeichnet, daß als komplexe Metallhydride Lithiumaluminiumhydrid, Natrium-, Kalium- oder Zinkborhydrid, Natriumcyanborhydrid oder Natriumborhydrid verwendet werden.

5. Verfahren nach einem der Ansprüche 1, 2 oder 3, dadurch gekennzeichnet, daß als komplexes Metallhydrid Natriumborhydrid verwendet wird.

6. Verfahren nach einem der Ansprüche 1 bis 5, dadurch gekennzeichnet, daß man die Reduktion in Wasser oder polaren organischen Lösungsmitteln bei Temperaturen von -20 bis +30°C vornimmt.

7. Verfahren nach einem der Ansprüche 1 bis 6, dadurch gekennzeichnet, daß
R₁ für einen gegebenenfalls substituierten C₁-C₁₂-Alkylrest, einen Cyclohexyl- oder Tetrahydronaphthyl-2-Rest oder einen gegebenenfalls substituierten Benzyl- oder Phenyl-Rest oder einen Indolyl-3-Rest steht.

8. Verfahren nach einem der Ansprüche 1 bis 6, dadurch gekennzeichnet, daß R₁ für Butyl und R₄ für einen Carbalkoxymethyl-Rest steht.

## Claims

1. Process for the stereoselective preparation of (S,S)- or (R,R)-β-amino alcohols of the formulae 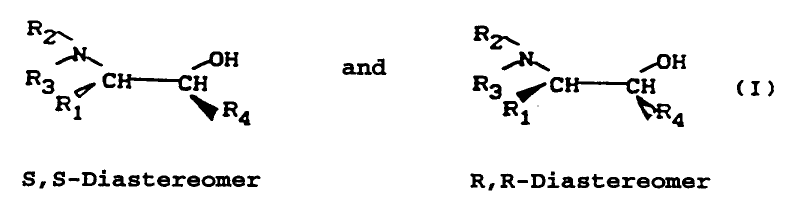 in which
R₁ represents an optionally substituted alkyl, cycloalkyl, alkenyl, aralkyl, aryl or heteroaryl radical,
R₂ and R₃ independently of one another denote an optionally substituted aralkyl group
and
R₄ represents an optionally substituted alkyl, alkenyl, alkinyl, aralkyl, aryl or heteroaryl radical,
characterized in that optically active α-amino ketones of the formulae 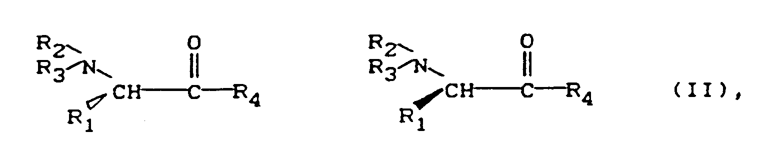 in which
R₁, R₂, R₃ and R₄ have the meaning given under formula (I), are reduced with complex metal hydrides.

2. Process according to Claim 1, characterized in that R₂ and R₃ independently of one another denote an optionally substituted benzyl group.

3. Process according to Claim 1, characterized in that R₂ and R₃ are unsubstituted benzyl radicals.

4. Process according to one of Claims 1, 2 or 3, characterized in that the complex metal hydrides used are lithium aluminium hydride, sodium, potassium or zinc borohydride, sodium cyanoborohydride or sodium borohydride.

5. Process according to one of Claims 1, 2 or 3, characterized in that the complex metal hydride used is sodium borohydride.

6. Process according to one of Claims 1 to 5, characterized in that the reduction is carried out in water or polar organic solvents at temperatures of -20 to +30°C.

7. Process according to one of Claims 1 to 6, characterized in that
R₁ represents an optionally substituted C₁-C₁₂-alkyl radical, a cyclohexyl or tetrahydronaphth-2-yl radical or an optionally substituted benzyl or phenyl radical or an indol-3-yl radical.

8. Process according to one of Claims 1 to 6, characterized in that R₁ represents butyl and R₄ represents a carbalkoxymethyl radical.

## Revendications

1. Procédé de production stéréosélective de (S,S)- ou (R,R)-β-aminoalcools de formules 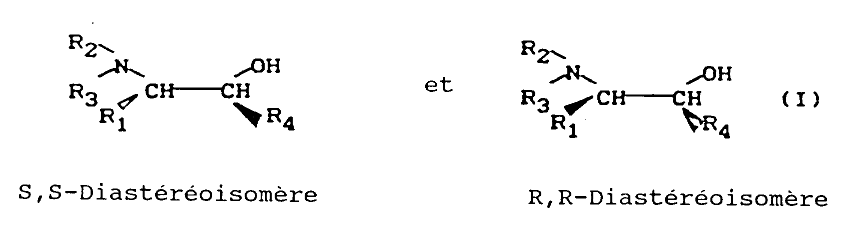 dans lesquelles
R₁ représente un reste alkyle, cycloalkyle, alcényle, aralkyle, aryle ou hétéroaryle éventuellement substitué,
R₂ et R₃ représentent, indépendamment l'un de l'autre, un groupe aralkyle éventuellement substitué et
R₄ est un reste alkyle, alcényle, alcynyle, aralkyle, aryle ou hétéroaryle éventuellement substitué,
caractérisé en ce qu'on réduit des α-aminocétones optiquement actives de formules 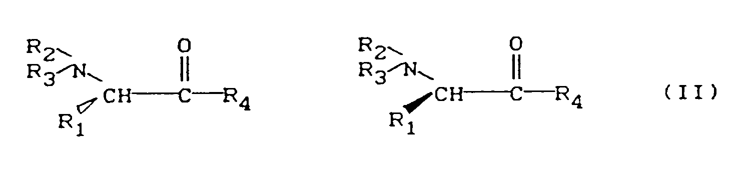 dans lesquelles
R₁, R₂, R₃ et R₄ ont la définition indiquée pour la formule (I), avec des hydrures métalliques complexes.

2. Procédé suivant la revendication 1, caractérisé en ce que R₂ et R₃ représentent, indépendamment l'un de l'autre, un groupe benzyle éventuellement substitué.

3. Procédé suivant la revendication 1, caractérisé en ce que R₂ et R₃ sont des restes benzyle non substitués.

4. Procédé suivant l'une des revendications 1, 2 et 3, caractérisé en ce qu'on utilise comme hydrures métalliques complexes l'hydrure de lithium et d'aluminium, le borohydrure de sodium, potassium ou zinc, le cyanoborohydrure de sodium ou le borohydrure de sodium.

5. Procédé suivant l'une des revendications 1, 2 et 3, caractérisé en ce qu'on utilise comme hydrures métalliques complexes le borohydrure de sodium.

6. Procédé suivant l'une des revendications 1 à 5, caractérisé en ce qu'on conduit la réduction dans l'eau ou dans des solvants organiques polaires, à des températures de -20 à +30°C.

7. Procédé suivant l'une des revendications 1 à 6, caractérisé en ce que
R₁ est un reste alkyle en C₁ à C₁₂ éventuellement substitué, un reste cyclohexyle ou tétrahydronaphtyle-2, ou un reste benzyle ou phényle éventuellement substitué, ou un reste indolyle-3.

8. Procédé suivant l'une des revendications 1 à 6, caractérisé en ce que R₁ est un reste butyle et R₄ est un reste carbalkoxyméthyle.
